# EUROPEAN PATENT APPLICATION

(11) **EP 4 268 675 A1**
(43) Date of publication of application: **01.11.2023**
(21) Application number: 21915206.3
(22) Date of filing: 23.12.2021
(51) Int. Cl.: A47C 27/00, A61G 7/043, A61B 5/0245, A61B 5/08, A61B 5/11, A61F 5/56

(54) **BEDDING SYSTEM**

(30) Priority: 28.12.2020 JP 2020218672
(71) Applicant: Nakatsugawa, Shigekazu, Tokyo, 134-0083 (JP)
(72) Inventor: Nakatsugawa, Shigekazu, Tokyo, 134-0083 (JP)
(74) Representative: Dehns
(86) International application number: PCT/JP2021/047966
(87) International publication number: WO 2022/145346

(57) **Abstract**

A bedding system includes: a bedding; an auxiliary mechanism that is incorporated in the bedding and is to assist change of a body position of the user, the auxiliary mechanism including a plurality of column members that are long along a thickness-wise direction of the bedding, are individually movable along the thickness-wise direction, and are arranged to be parallel to each other; and a first control unit that controls movement of each of the column members.

## Description

### Technical Field

The present invention relates to a bedding system to be used by a user during sleep.

### Background Art

In recent years, the situations surrounding humankind are changing drastically: extreme climates due to environmental destruction associated with global warming; widening disparities in all aspects due to the global economy; possibility of war due to the intensifying conflict between the U.S. and China; fear of death, especially among the elderly and those with chronic illnesses, due to the worldwide spread of the COVID-19, and so on, all the above situations are rapidly advancing on a global scale. The disparities are not limited to economic power, nutrition, and education, and widely extend to abilities of exercise, information acquisition and processing, and the like. In other words, it is not an exaggeration to say that humankind is in a vortex of unsurpassed violent motion. In such situations, it is quite difficult for people to maintain good sleep that is sufficient in both quantity and quality which are important for maintaining peace of mind and physical health. On the other hand, it has been considered that with aging, sleep, like other vital functions such as reduced physical activity, undergoes changes, so that it is inevitable that sleep time, which is strongly correlated with cognitive function, becomes shorter, shallow sleep increases, and deep sleep decreases. That is, it has been considered that the risk of dementia increases with aging and the shortening of the healthy life span is unavoidable.

The life (daily activity) of people consists of social activity outside the home accounting for about 34 to 50% inclusive in length, home life accounting for about 17 to 33% inclusive in length, and sleep time accounting for the remaining about 33% in length, and is controlled by a biological clock. During sleep, memory is fixed, and sleep plays a major role in maintenance of visceral functions, and it has become clear that sleep is indispensable for improving productivity. In a sense, activities other than sleep time are supported by psychology and physical environments, etc., that is, water intake, nutrition (reflecting digestion, absorption, metabolism, excretion, etc.), exercise, sleep, lifestyle habits, memory, purpose of life, etc., and conversely, sleep reflects physical or psychological fatigue, stress, psychological state, other social activities, nutrition, exercise, lifestyle habits, etc., and its quality and quantity are influenced.

It is also known that sleep varies greatly among individuals in terms of change in body temperature, balance between calorific values (exercise amount, meal content, etc.) and heat emission amounts (perspiration ability, etc.), etc., and is also greatly affected by the climate of the living area, that is, the environment such as temperature, moisture, sound, light, oxygen saturation, carbon dioxide concentration, and carbon monoxide concentration. With regard to individual differences, there are wide and large differences in individual body composition, particularly muscle mass related to calorific value, cardiovascular system / nervous system related to skeleton / heat dissipation / perspiration, development of internal organs / degree of aging, combination of lifestyle-related diseases, immune / endocrine system balance or the like, and quality / content of work, etc. Actually, the sensible comfortable temperature greatly depends on the moisture even when viewed from the wet bulb globe temperature.

By the way, the temperature of each body part is determined by a balance of firstly, factors that lead to a temperature rise:
1 import of heat (mainly by arterial blood);
2 thermogenesis (mainly muscle and internal organs (e.g., liver) and hyperemia, inflammatory response);
3 thermal convection (transfer of heat from a hot gas (or liquid) passing over exposed skin to the skin);
4 thermal conduction (transfer of heat from a surface (solid) that is in direct contact and is hotter than body temperature to a colder body surface);
5 suppression of thermal convection and conduction (obesity (reduction in body surface area), thick wear, wind shielding),

   secondly, factors that lead to a temperature drop:
6 heat emission radiation (mainly affected by infrared rays, body surface area (thinness), and autonomic nerve balance to reduce the temperature difference from the surrounding environment);
7 evaporation (e.g., cooling by evaporation of sweat or moisture);
8 thermal convection (transfer of heat to a cold gas (or liquid) passing over exposed skin, for example, ventilation);
9 thermal conduction (conduction of heat from the body surface to a surface (solid) cooler than the body surface temperature, or transfer of heat from a high-temperature surface in direct contact to a low-temperature (e.g., ischemic site) surface);
10 export of heat (mainly by venous blood / cutaneous vein / capillary blood, and ischemia), and so on.

Thermogenesis is due to muscle exercise (60%) and visceral metabolism (40%). In addition, sympathetic excitation, hyperthyroidism, severe obesity, and the like also affect the rise in body temperature. On the other hand, in the cases of parasympathetic nerve dominance, hypothyroidism, and wasting, the body temperature decreases.

Although 30 minutes has conventionally been assumed physiologically as one unit of sleep, this may be correlated with the rhythm of turning over which is considered to be once in about 30 minutes, and of course, individual differences are recognized. On the other hand, in addition to conventional sleep disorders associated with child-care, 24-hour services such as medical services, nursing services, production services, distribution services, commercial services, traffic services, security services, and global services, which cannot be adjusted at all by a biological clock, have become common nowadays, and those services including shift work cause serious sleep disorders. In particular, alcohol consumption by pilots has become a major social problem, and the problems of shift work ignoring physiological biological clock, continuous work for nearly 24 hours, and time difference have a high risk of inducing alcohol dependence, drug dependence, and the like. Although not a service, child-care may also cause serious sleep disorders.

It has also been found that frailty and at least a part of various lifestyle-related diseases associated with extreme eating disorders, wasting, and severe obesity, which have become problems in developed countries, correlate with problems such as sleep quality, and that sleep disorders are related to exacerbation of obesity and some lifestyle-related diseases in addition to depression, etc. For example, in sleep apnea syndrome, it is known that subjective symptoms related to sleep vary greatly among individuals, such as a case where there are serious objective signs even when the AHI (apnea hypopnea index) is the same and a case where there is no extremely serious subjective symptom at all..

In Japan, before the spread of the COVID-19 infection, the ratio of part-time employment work to employment has become high, there are not a few stressful workplace environments, and it is difficult to obtain a stable environment because Japan has a long land extending from north to south and has four seasons, and thus in Japan, the sleep time is the shortest as compared with other developed countries, and the ratio of those who are not satisfied with sleep such as having sleep disorders is said to be higher than in other countries. In monsoon regions such as Japan, the humidity is originally high except during periods such as the winter season, and the comfortable temperature range for sleep is extremely narrow even when viewed from the wet bulb globe temperature, and such conditions may also be a cause of sleep disorders. In addition to arteriosclerosis, the aging of the population has inevitably led to the progression of osteoarthritis of the spine, which has various effects on the balance regulation of the autonomic nervous system. In particular, the head and neck are the most sensitive parts because they have a dense distribution of sensory nerves as well as central nerves such as the brain and spinal cord including the sleep center. The carotid sinus also has a central role in monitoring various changes in the blood, highlighting the specificity of neck related to sleep.

Furthermore, as described above, the number of people who are not able to freely perform turning over, which is important for sleep, is increasing because of difficulty in supporting their body weight due to lack of muscles (sarcopenia) in the case of severe obesity or wasting associated with lack of exercise during aging. As described above, the autonomous thermoregulatory function decreases with aging. When a person is in a supine position during sleep, the base of the tongue tends to fall back and the airway tends to narrow, and when the person is in a lateral position, the airway tends to be secured, and thus, turning over is necessary for comfortable sleep. In the case of obesity, deposition of subcutaneous fat also progresses in the neck, and thus, the distance between the skin surface and the artery and vein increases, heat dissipation decreases, the skin stretches, the distribution of sweat glands per unit area decreases, and the sweating ability of the neck decreases, thereby preventing a decrease in the temperature of the brain, which is necessary for sleep. Under such circumstances, sudden deaths due to arrhythmias caused by sleep apnea syndrome or pulmonary infarction caused by economy class syndrome, etc. are also increasing.

On the other hand, it has become known that sleep and the neuroendocrine system related thereto are also involved in the metabolism of various neurotransmitters in the brain and individual differences are not small in complicated pathological conditions, experience, smoking or nonsmoking, contents of coffee intake, exercise, contents of meal, living habits affecting autonomic nerves such as bathing or showering, etc. Therefore, various drugs have been developed and applied widely for sleep disorders and the like, but there are individual differences in efficacy and dependence on many of the hypnotics has been recognized, and in recent years, their long-term use has been suppressed.

In addition, it is known that when the sleep time itself is shorter than 5 hours, when the nap is 30 minutes or more, and when there is a sleep apnea syndrome, etc., the risk of lifestyle-related diseases such as diabetes and sleep disorders is high, and the importance of the regulation mechanism of the autonomic nerves related to the biological clock has become known, and the importance of ensuring deep sleep time with sustained nasal breathing, which can make the parasympathetic nerves dominant, has been suggested. In this sense, it can be said that know-how for realizing comfortable sleep including a new therapy for sleep apnea syndrome (therapy for upper-airway restoration and maintenance, hereinafter referred to as "TURM") is necessary in an era of unavoidable anxiety called living with COVID-19. On the other hand, it is known that extremely strong sleepiness due to hypothermia occurs without individual differences in accidents, etc. such as mountain climbing accidents.

Techniques for providing optimal sleep have been proposed heretofore. For example, Patent Literature 1 discloses a bed system for assisting a user in changing the body position during sleep. Specifically, according to Patent Literature 1, the bed bottom is divided into two, i.e., a left half and a right half, and both the left half and the right half are inclined to assist the user to be in a lateral position.

### Citation List

### Patent Literature

Patent Literature 1: JP 2016-067429 A

### Summary of Invention

### Technical Problem

According to the technique of Patent Literature 1, the left half region and the right half region of the bed bottom can be respectively inclined only in a planar manner. In other words, the bed bottom cannot be moved for each subdivided region. In addition, in the configuration in which the bed bottom is inclined in a planar manner, the shape of the bed bottom does not match the body surface shape of each body part. Therefore, the configuration cannot avoid discomfort caused by the fact that unevenness occurs in the body weight load for each part and a body weight load of several times to ten times or more occurs in a limited part in the body every time the center of gravity moves. As described above, it is difficult to appropriately assist the user in changing the body position during sleep. In view of the above circumstances, an object of the present invention is to provide a bedding system that appropriately assists a user in changing the body position at any time during sleep.

### Solution to Problem

To solve the above-described problem, the bedding system according to the present invention includes: a bedding; an auxiliary mechanism that is incorporated in the bedding and is to assist change of a body position of the user, the auxiliary mechanism including a plurality of column members that are long along a thickness-wise direction of the bedding, are individually movable along the thickness-wise direction, and are arranged to be parallel to each other; and a first control unit that controls movement of each of the column members.

### Advantageous Effects of Invention

According to the bedding system of a preferred aspect of the present invention, since each of the column members is individually movable along the thickness-wise direction of the bedding, it is possible to move the bedding in each region corresponding to each of the column members. Therefore, it is possible to assist the user during sleep in appropriately changing the body position (specifically, comfortably turn over). That is, it is possible to provide sleep optimized for each user. Consequently, without depending on chemicals or drugs and by a physical or environmental approach with relatively little individual differences, it is possible to achieve individualized sleep optimization, to radically improve the sleep quality of individuals, and to improve the productivity of activities other than sleep while preventing various diseases including infections. In addition, by realizing comfortable sleep, the cognitive function can be maintained and the healthy life span can be extended.

### Brief Description of Drawings

Fig. 1 is a plan view of a bedding system according to a first embodiment.
Fig. 2 is a side view of a mattress.
Fig. 3 is a side view of the mattress.
Fig. 4 is a block diagram illustrating a function of the mattress.
Fig. 5 is an explanatory view illustrating a movement of a column member.
Fig. 6 is an explanatory view illustrating a movement of the column member.
Fig. 7 is an explanatory view illustrating a movement of the column member.
Fig. 8 is an explanatory view illustrating a movement of the column member.
Fig. 9 is a plan view of a bedding system according to a second embodiment.
Fig. 10 is a side view of a pillow.
Fig. 11 is an explanatory view illustrating a movement of a column member.
Fig. 12 is an explanatory view illustrating a movement of the column member.
Fig. 13 is an explanatory view illustrating a movement of the column member.
Fig. 14 is an explanatory view illustrating a movement of the column member.

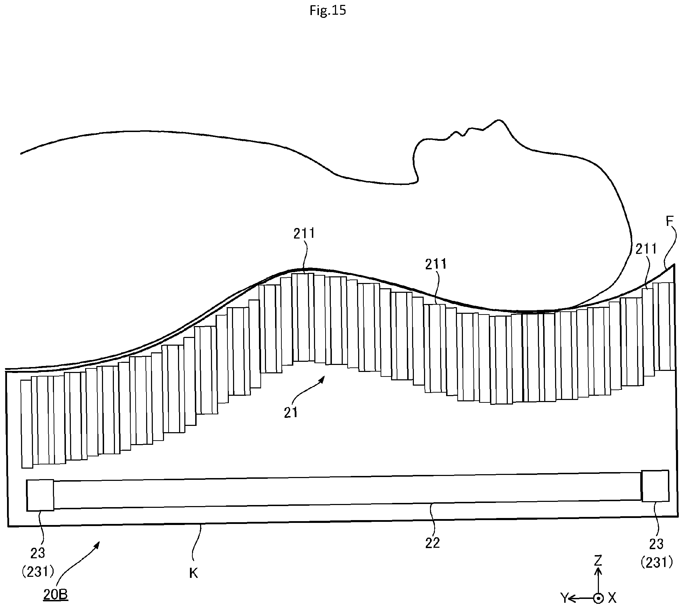

Fig. 15 is a side view of a pillow according to another aspect of the second embodiment.
Fig. 16 is a configuration diagram of a moisture mechanism according to a modification.

### Description of Embodiments

### <First embodiment>

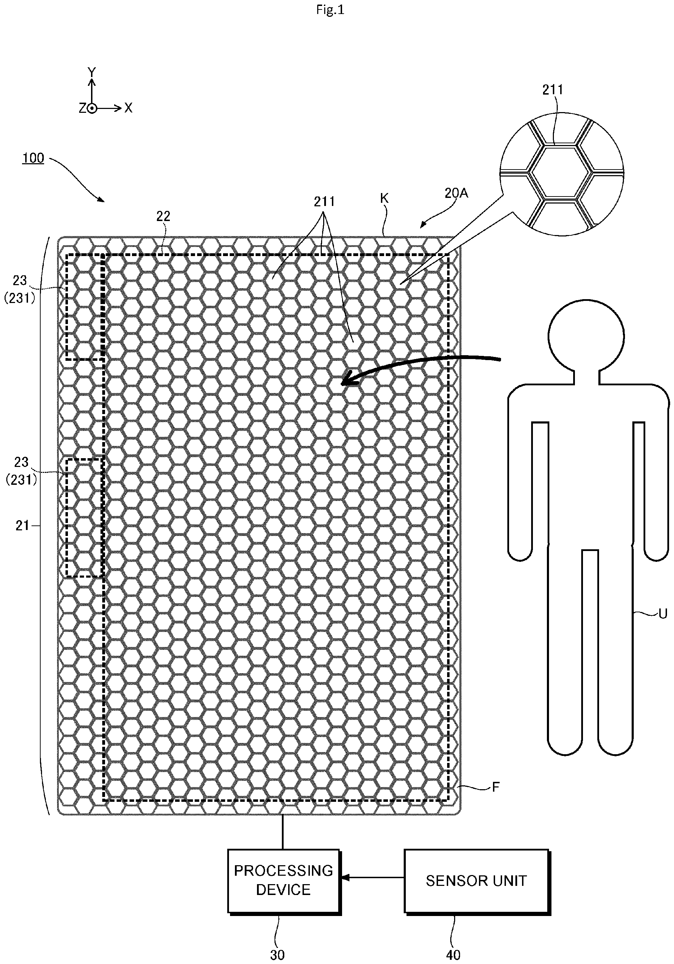

Fig. 1 is a plan view of a bedding system 100 according to a first embodiment. The bedding system 100 is related to bedding for assisting sleep of a user U. As illustrated in Fig. 1, the bedding system 100 includes a mattress 20A (an example of "bedding"), an auxiliary mechanism 21, a temperature mechanism 22, a moisture mechanism 23, a processing device 30, and a sensor unit 40. According to the first embodiment, the mattress 20A on which the user U lies during sleep is illustrated as bedding.

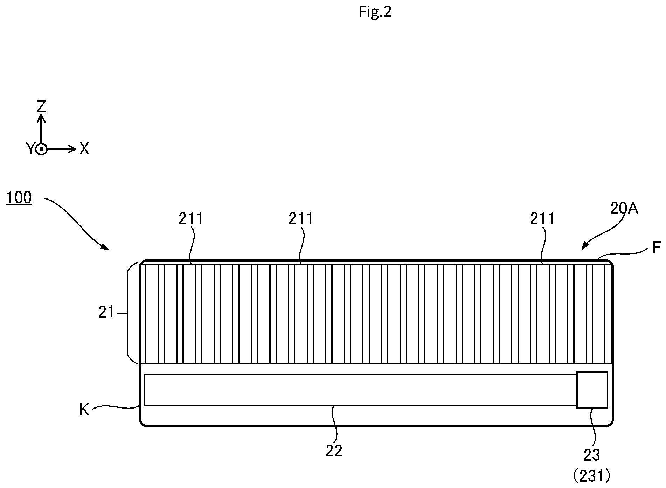

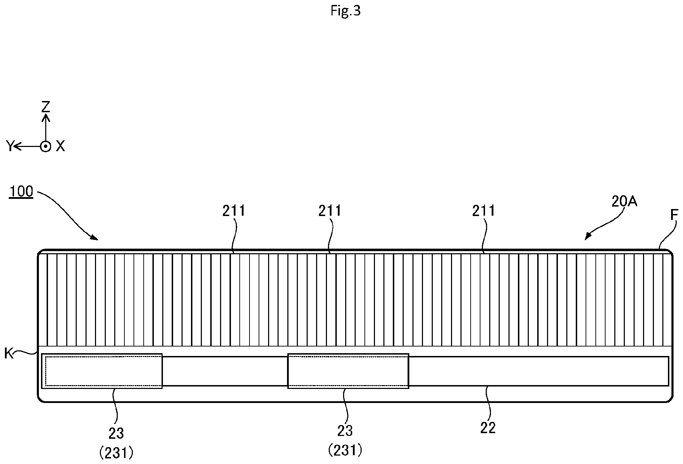

In the following description, a thickness-wise direction of the mattress 20A is referred to as a Z direction, a direction orthogonal to the Z direction is referred to as an X direction, and a direction orthogonal to the Z direction and the X direction is referred to as a Y direction. In Fig. 1, the lateral direction of the mattress 20A is the X direction, and the longitudinal direction of the mattress 20A is the Y direction. Fig. 2 is a side view of the mattress 20Awhen viewed from the Y direction, and Fig. 3 is a side view of the mattress 20A when viewed from the X direction.

The mattress 20A includes, for example, a container K. The container K is formed of, for example, an elastic material. The container K includes a surface (hereinafter referred to as "contact surface") F on which the user U lies. The contact surface F is a surface of the mattress 20A on the positive side in the Z direction.

As illustrated in Figs. 1 to 3, the auxiliary mechanism 21, the temperature mechanism 22, and the moisture mechanism 23 are incorporated in the mattress 20A (container K). Although the auxiliary mechanism 21, the temperature mechanism 22, and the moisture mechanism 23 are not actually exposed on the outer surface of the mattress 20A, the auxiliary mechanism 21, the temperature mechanism 22, and the moisture mechanism 23 are illustrated in Figs. 1 to 3 for convenience. The mattress 20A can withstand a load of about 350 kg in a state in which the auxiliary mechanism 21, the temperature mechanism 22, and the moisture mechanism 23 are incorporated.

### <Sensor unit 40>

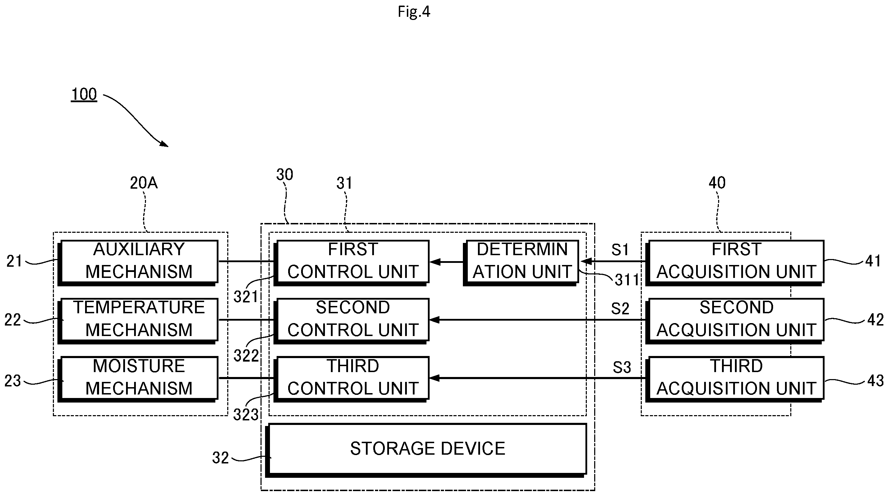

Fig. 4 is a block diagram illustrating a function of the bedding system 100 according to the first embodiment. As illustrated in Fig. 4, the sensor unit 40 is a detection device that generates a detection signal S (S1, S2, S3) for identifying information related to a living body of the user U (hereinafter referred to as "biological information"). The sensor unit 40 of the first embodiment includes a first acquisition unit 41, a second acquisition unit 42, and a third acquisition unit 43.

The first acquisition unit 41 is a sensor that generates a detection signal S1 that indicates a displacement in accordance with the load of the user U on the contact surface F. For example, a piezoelectric element may be illustrated as the first acquisition unit 41. The detection signal S1 is used to identify the position of the user U on the mattress 20A (contact surface F) (hereinafter referred to as "body position") and the period that has elapsed since the user U most recently changed the body position (hereinafter referred to as "change period"). For example, a plurality of the first acquisition units 41 may be provided at predetermined intervals in a region on the contact surface F side inside the mattress 20A (container K). Here, the number of the first acquisition units 41 is arbitrary.

The detection signal S1 generated by the first acquisition unit 41 corresponding to the position of the user U on the contact surface F among the plurality of the first acquisition units 41 varies. Thus, it is possible to estimate the body position depending on the detection signal S1. In addition, since the detection signal S1 generated by the first acquisition unit 41 changes when the user U moves, it is also possible to detect a change of the body position of the user U. The body position and the change period are examples of biological information.

Any sensor may be used as the first acquisition unit 41 as long as the body position and the change period can be identified. For example, a radio wave sensor, an acceleration sensor, or an optical sensor may be used as the first acquisition unit 41. The first acquisition unit 41 continuously generates the detection signal S1. That is, a change over time in the load of the user U is detected.

The second acquisition unit 42 is a sensor that generates a detection signal S2 representing the body temperature of the user U. The detection signal S2 is used to identify the body temperature (an example of biological information) of the user U. For example, any known body temperature sensor (for example, an infrared sensor) may be used as the second acquisition unit 42. The second acquisition unit 42 is mounted (attached) to, for example, the body surfaces of a plurality of parts (trunk, both hands, both feet, head, neck, and the like) of the body of the user U. A non-contact type body temperature sensor may be used as the second acquisition unit 42 as long as the detection signal S2 representing the body temperature of the user U can be generated. The second acquisition unit 42 continuously generates the detection signal S2.

The third acquisition unit 43 is a sensor that generates a detection signal S3 representing the moisture of the body surface of the user U. The detection signal S3 is used to identify the moisture of the body surface (an example of biological information) of the user U. For example, any known moisture sensor may be used as the third acquisition unit 43. For example, the third acquisition unit 43 may be mounted to the same position as the second acquisition unit 42 on the body surface of the user U. A non-contact type moisture sensor may be used as the third acquisition unit 43 as long as the detection signal S3 representing the moisture of the body surface of the user U can be generated. In addition, a device in which the second acquisition unit 42 and the third acquisition unit 43 are integrated may be used. The second acquisition unit 42 continuously generates the detection signal S2. The number of the second acquisition units 42 and the number of the third acquisition units 43 are arbitrary.

### <Auxiliary mechanism 21>

The auxiliary mechanism 21 is a mechanism for assisting change of the body position of the user U lying on the mattress 20A (typically, turning over of the user U during sleep). Specifically, the auxiliary mechanism 21 includes a plurality of column members 211. Each of the column members 211 is a columnar member that is long along the Z direction (the thickness-wise direction of the mattress 20A). The plurality of the column members 211 are closely arranged in a manner of being parallel to each other. The plurality of the column members 211 are arranged in a manner of extending over the entire mattress 20A in a plan view.

As illustrated in Fig. 1, the column member 211 is, for example, a regular hexagonal prism. The diameter of the column member 211 (the length of the diagonal line of the regular hexagon) is, for example, 20 to 40 mm inclusive. The column member 211 is not limited to a regular hexagonal prism. For example, a polygonal prism (e.g., a quadrangular prism) other than a circular cylinder or a regular hexagonal prism may be used as the column member 211. The column member 211 of the first embodiment is hollow. In other words, the column member 211 is tubular. That is, a through-hole along the Z direction is formed in the column member 211.

The plurality of the column members 211 are individually movable along the Z direction. Specifically, each of the column members 211 is movable from a predetermined position (initial position) to the positive side and the negative side of the Z direction (in the vertical direction) respectively within a predetermined range. Each of the column members 211 is movable so that the body position of the user U can be changed. Any of various actuators may be used as appropriate to move each of the column members 211. The column member 211 may be formed of any material that can be made lightweight and is durable.

The movement of each of the column members 211 of the auxiliary mechanism 21 is controlled in accordance with the biological information. According to the first embodiment, the movement of the column member 211 is controlled in accordance with the body position and the change period. That is, according to the first embodiment, the auxiliary mechanism 21 is controlled in accordance with two pieces of biological information. The change of the body position is typically turning over.

### <Temperature mechanism 22>

The temperature mechanism 22 is a mechanism for adjusting the temperature of the mattress 20A. Therefore, it is possible to adjust the body temperature of the user U. For example, the temperature mechanism 22 may include a liquid whose temperature can be changed (hereinafter referred to as "adjustment liquid") and an adjustment device 213. The entire inside of the container K is filled with the adjustment liquid. For example, the adjustment liquid may be filled in the gap between the column members 211, the inside of the column member 211, the upper side and the lower side of the column member 211, and the like. Therefore, each of the column members 211 can be moved in the vertical direction and the lateral direction by buoyancy. The adjustment liquid may be filled in the container K in any manner. For example, a tube filled with the adjustment liquid, a bag made of resin and filled with the adjustment liquid, or the like may be contained in the container K.

The adjustment device 213 includes a temperature adjustment unit (not illustrated) and a circulation unit (not illustrated). For example, the adjustment device 213 may be positioned below the column member 211 (on the positive side in the Z direction). The temperature adjustment unit is a device capable of changing (cooling or heating) the temperature of the adjustment liquid. For example, the temperature adjustment unit may be configured by a plurality of Peltier elements capable of cooling the adjustment liquid and a heater capable of heating the adjustment liquid. The temperature of the adjustment liquid is changed by bringing the temperature adjustment unit into indirect contact with the adjustment liquid. The circulation unit (for example, a pump) is a device that circulates the adjustment liquid in a manner of circulating the adjustment liquid inside the container K. It is preferable that the temperature mechanism 22 of the mattress 20A is configured to capable of adjusting the temperature for each of a plurality of regions corresponding respectively to a plurality of parts (trunk, four limbs, neck, and head) of the body of the user U. The specific configuration of the temperature mechanism 22 is arbitrary as long as the temperature of the mattress 20A can be adjusted.

The temperature mechanism 22 is controlled in accordance with the biological information. According to the first embodiment, the temperature mechanism 22 is controlled in accordance with the body temperature of the user U. A part of the temperature mechanism 22 (for example, the adjustment device 213) may be provided outside the mattress 20A.

### <Moisture mechanism 23>

The moisture mechanism 23 is a mechanism for absorbing or increasing moisture. Specifically, the moisture mechanism 23 includes a plurality of air blowing devices (for example, compressors or fans) capable of blowing air whose moisture is adjusted (dehumidified or humidified). The moisture mechanism 23 is installed at an arbitrary position inside the container K. For example, the air blowing devices may be installed at a position where the blown air hits the user U from the contact surface. Therefore, it is possible to adjust the moisture of the body surface of the user U by the air blown from the moisture mechanism 23. It is preferable that the moisture mechanism 23 of the mattress 20A is configured to be capable of adjusting moisture for each of a plurality of parts of the body of the user U. The specific configuration of the moisture mechanism 23 is arbitrary as long as the moisture of the mattress 20A can be adjusted.

The moisture mechanism 23 is controlled in accordance with the biological information. According to the first embodiment, the moisture mechanism 23 is controlled in accordance with the moisture of the body surface of the user U. The moisture mechanism 23 may be provided outside the mattress 20A.

### <Processing device 30>

The processing device 30 of Fig. 4 is a device that controls the auxiliary mechanism 21, the temperature mechanism 22, and the moisture mechanism 23. The processing device 30 of the first embodiment is realized by a computer system including a control device 31 and a storage device 32. For example, an information terminal such as a personal computer or a tablet may be used as the processing device 30.

The control device 31 is, for example, configured of a single or a plurality of processing circuits such as CPUs (Central Processing Units), and integrally controls each element of the processing device 30. The storage device 32 is, for example, a single or a plurality of memories configured of a known recording medium such as a magnetic recording medium or a semiconductor recording medium, and stores a program executed by the control device 31 and various data used by the control device 31. As illustrated in Fig. 4, the control device 31 of the first embodiment functions as a determination unit 311, a first control unit 321, a second control unit 322, and a third control unit 323.

The determination unit 311 determines whether or not the change period exceeds a predetermined threshold Z. That is, it is determined whether or not a predetermined period (threshold Z) has elapsed since the user U most recently changed the body position (turned over). The change period is identified from the detection signal S1 generated by the first acquisition unit 41.

The first control unit 321 controls the movement of each of the column members 211 of the auxiliary mechanism 21 in accordance with the biological information (the change period and the body position). Specifically, the timing at which each of the column members 211 moves and the position to which each of the column members 211 moves are controlled in accordance with the biological information.

First, the first control unit 321 controls the timing at which each of the column members 211 moves in accordance with the change period. Specifically, when the determination unit 311 determines that the change period exceeds the threshold Z, the first control unit 321 causes each of the column members 211 to move. That is, when the body position has not been changed for a predetermined period of time, each of the column members 211 moves (the body position of the user U is changed). The threshold Z is set to, for example, 20 to 30 minutes inclusive from the viewpoint that it is effective to turn over every about 30 minutes to prevent snoring, sleep apnea, congestion, muscle stiffness, and the like.

Second, the first control unit 321 controls the position to which each of the column members 211 moves in accordance with the body position. The body position is estimated depending on the detection signal S1 generated by the first acquisition unit 41. The body position is, for example, the position of the body axis of the user U. Specifically, the first control unit 321 controls the position of each of the column members 211 so as to turn the body over to a position different from the current body position.

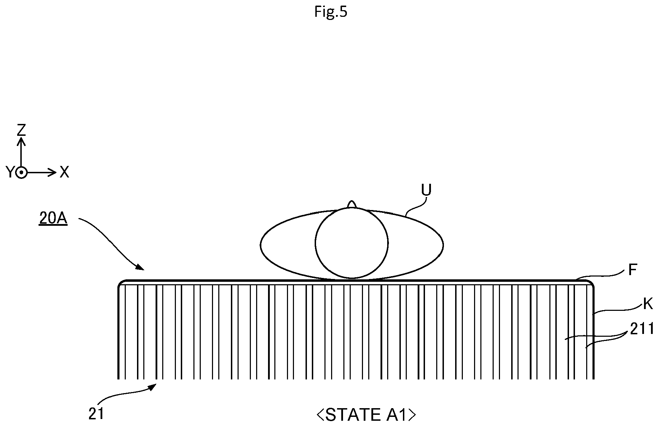

Hereinafter, an example of the movement of the plurality of the column members 211 of the auxiliary mechanism 21 will be described. Figs. 5 to 8 are explanatory views illustrating an example of the movement of the column members 211 when viewed from the Y direction. Figs. 5 to 8 show a state of the column members 211 when the user U changes the body position (turns over) from a supine position to a lateral position.

As illustrated in Figs. 5 to 8, for example, the movement of the column members 211 is controlled so as to transition from a state A1 to a state A2 to a state A3 to a state A4. As a result, the user U changes the body position (turns over) from the supine position to the lateral position. The body position is continuously identified from the state A1 to the state A4. The movement of the column members 211 from the state A1 to the state A4 is performed by identifying the body position.

Schematically, the user U is caused to turn over by lowering the tip (the end portion on the positive side in the Z direction) of one or more of the column members 211 corresponding to one of the left and right sides of the user U when viewed from the Y direction among the plurality of the column members 211.

The state A1 in Fig. 5 is a state of the column members 211 when the user U is in the supine position. As illustrated in Fig. 5, in the state A1, for example, the positions of the tips of all the column members 211 in the Z direction are the same. In Fig. 5, a case where the positions of the tips of all the column members 211 in the Z direction are the same is illustrated as the state A1. Here, in the state A1, for example, the tips of the column members 211 may be located at different positions depending on the body of the user U so as not to burden the body.

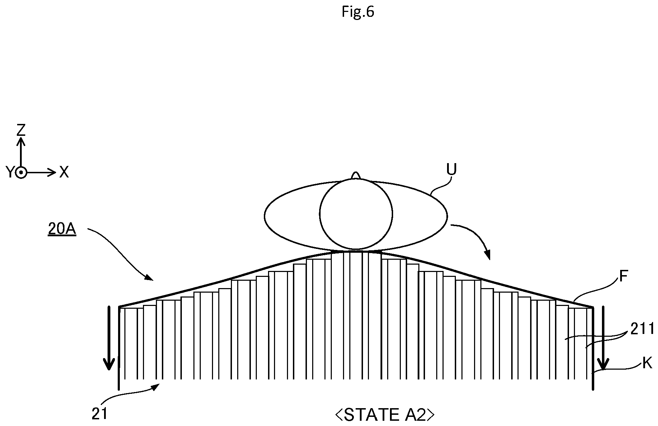

In the state A2 of Fig. 6, among the plurality of the column members 211, one or more of the column members 211 corresponding to predetermined widths (lengths in the X direction) on both the left and right sides (negative side and positive side in the X direction) of the body position of the user U are lowered (moved to the negative side in the Z direction). As illustrated in Fig. 6, for example, the column members 211 are moved so as to incline the region from the body position side to the end portion on the positive side and the end portion on the negative side in the X direction. Among the plurality of the column members 211, the column members 211 positioned at the end portion on the positive side and the end portion on the negative side in the X direction have, for example, the tips lowered by about 50 mm as compared with the column members 211 corresponding to the body position. In the state A1, when the determination unit 311 determines that the change period exceeds the threshold Z, the operation of moving the plurality of the column members 211 from the state A2 to the state A4 is executed.

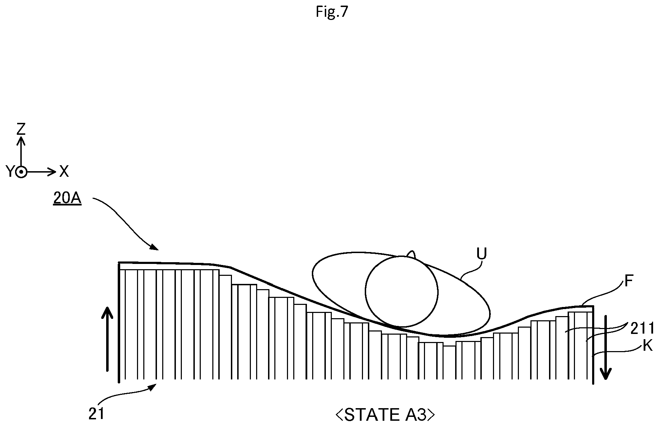

In the state A3 of Fig. 7, one or more of the column members 211 positioned on one of the left and right sides of the user U among the plurality of the column members 211 are further lowered. Specifically, after the state A2 is set, the column members 211 on the side to which the user U voluntarily moves are further lowered. Fig. 7 illustrates a case where one or more of the column members 211 positioned on the right side (positive side in the X direction) of the user U are further lowered from the state A2. For example, the end portion on the positive side in the X direction is lowered by about 50 mm from the state A2.

As illustrated in Fig. 7, the column members 211 may be moved so that the position corresponding to the right shoulder part of the user U is the lowest. On the other hand, one or more of the column members 211 positioned on the left side (negative side in the X direction) of the user U are raised from the state A2 (for example, raised by about 100 mm). As long as the end portion on the positive side in the X direction is within 60 mm from the state A2, the distance to be lowered is arbitrary. Similarly, as long as the end portion on the positive side in the X direction is within 60 mm from the state A2, the distance to be raised is arbitrary.

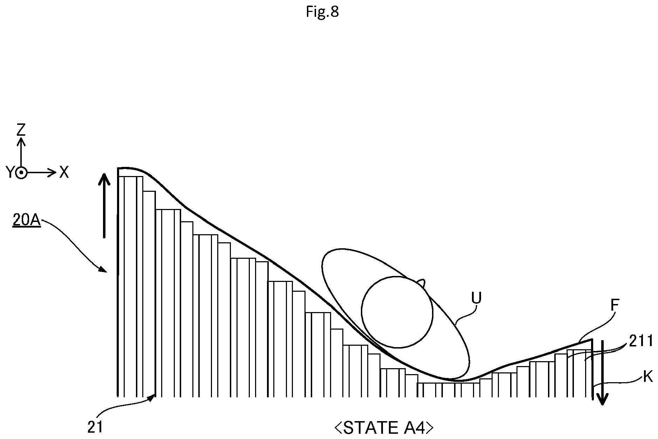

In the state A4 of Fig. 8, from the state A3, one or more of the column members 211 positioned on the right side of the user U are further lowered, and one or more of the column members 211 positioned on the left side of the user U are further raised. For example, in the state A4, from the state A1, one or more of the column members 211 positioned on the right side of the user U are lowered by about 60 to 100 mm inclusive, and one or more of the column members 211 positioned on the left side of the user U are raised by about 60 to 100 mm inclusive. That is, a height difference of about 120 to 200 mm inclusive is generated in the plurality of the column members 211.

Due to the movement from the state A1 to the state A4, among the plurality of the column members 211, one or more of the column members 211 on one of the left and right sides of the user U (the right side in the first embodiment) when viewed from the Y direction move downward, and one or more of the column members 211 on the other side (the left side in the first embodiment) move upward. That is, among the plurality of the column members 211, the inclination from one side to the other side of the user U when viewed from the Y direction has a gentle gradient. Since the body position of the user U is changed in accordance with the movement of the plurality of the column members 211, it is possible to change the body position (turn over) from the supine position to the lateral position.

Among the plurality of the column members 211, one or more of the column members 211 on the side to be lowered (left side or right side) within a width (about 400 mm) that exceeds the shoulder width on the side to be lowered when viewed from the Y direction are lowered. On the other hand, it is preferable that among the plurality of the column members 211, the side to be raised when viewed from the Y direction has a width (length in the X direction) as small as possible.

A specific example of the movement of the column members 211 in a case where the body position of the user U is changed from the supine position to the lateral position is not limited to the above. The method of moving the column members 211 is arbitrary as long as it is possible to cause the user U to turn over by lowering the tip of one or more of the column members 211 on one of the left and right sides of the user U when viewed from the Y direction among the plurality of the column members 211.

In addition, in the first embodiment, the case where the body position of the user U is changed from the supine position to the lateral position is illustrated, but the change of the body position is not limited to the above example. For example, the column members 211 may be moved so that the user U changes from the supine position to the lateral position, or the column members 211 may be moved so that the user U changes from one supine position to another supine position.

The second control unit 322 controls the temperature mechanism 22 in accordance with the body temperature of the user U (biological information). Hereinafter, an example of a method in which the second control unit 322 controls the temperature mechanism 22 will be described, but the method of controlling the temperature mechanism 22 is not limited to the example.

For example, the second control unit 322 controls the temperature mechanism 22 so that the body temperature of each body part (trunk, four limbs, neck, and head) is maintained at a body temperature which is a target (hereinafter referred to as "target body temperature"). The body temperature of each body part is identified from the detection signal S2 acquired by the second acquisition unit 42. For example, when there is no lesion such as inflammation in each body part, the second control unit 322 controls the temperature mechanism 22 so that the target body temperature is 26.0°C for the trunk and the four limbs, 25.0°C for the neck, and 18.0°C for the head, and then controls the temperature mechanism 22 so that the target body temperature of each part rises (+0.25 to 0.5°C inclusive per hour) over time. Here, it is not necessary to control the temperature mechanism 22 so that the target body temperature is changed over time. When the body temperature identified using the detection signal S2 is lower than the target body temperature, the temperature of the adjustment liquid of the temperature mechanism 22 is controlled to become higher, and when the body temperature identified using the detection signal S2 is higher than the target body temperature, the temperature of the adjustment liquid of the temperature mechanism 22 is controlled to become lower.

The third control unit 323 controls the moisture mechanism 23 in accordance with the moisture of the body surface of the user U. Hereinafter, an example of a method in which the third control unit 323 controls the moisture mechanism 23 will be described, but the method of controlling the moisture mechanism 23 is not limited to the example.

For example, the third control unit 323 controls the temperature mechanism 22 so that the moisture of the body surface of each body part (trunk, four limbs, neck, and head) is maintained at a moisture which is a target (hereinafter referred to as "target moisture"). The moisture of each body part is identified from the detection signal S3 acquired by the third acquisition unit 43. For example, when there is no lesion such as inflammation in each body part, the third control unit 323 controls the moisture mechanism 23 so that the target moisture of each body part is 50% or less. When the moisture of the body surface identified using the detection signal S3 is lower than the target moisture, the moisture mechanism 23 is controlled so as to blow humid air, and when the moisture identified using the detection signal S3 is higher than the target moisture, the moisture mechanism 23 is controlled so as to blow dry air.

The method of setting the target body temperature and the target moisture is arbitrary. For example, the user U himself / herself may set the target body temperature and the target moisture for each part by input using an input device (not illustrated) of the processing device 30. In addition, the target body temperature and the target moisture may be changed over time, for example.

As understood from the above description, according to the first embodiment, since each of the plurality of the column members 211 is individually moved, it is possible to move the mattress 20A in each region corresponding to each of the column members 211. That is, it is possible to move the mattress 20A by a subdivided region unit. Therefore, it is possible to appropriately assist the user U in changing the body position during sleep. Consequently, it is possible to change the body position of the user U without making the user U take an unnatural posture.

According to the bedding system 100 of the first embodiment, in particular, since the movement of the plurality of the column members 211 is controlled using the biological information, it is possible to assist the change of the body position according to the user U. That is, it is possible to provide sleep optimized for each user U.

According to the first embodiment, since the movement of the plurality of the column members 211 is controlled in accordance with the body position and the change period, it is possible to individually assist the change of the optimum body position for each user.

Further, since the temperature mechanism 22 and the moisture mechanism 23 are incorporated in the mattress 20A, for example, when it is difficult to appropriately maintain the temperature and the moisture in the room, it is also possible to appropriately maintain the body temperature and the moisture of the user U. That is, it is possible to appropriately assist the user during sleep to change the body position at any time while adjusting the temperature and moisture. As can be understood from the above description, according to the bedding system 100 of the first embodiment, it is possible to provide the user with comfortable sleep.

<Second embodiment>

A second embodiment of the present invention will be described. Elements having the same actions or functions as those of the first embodiment in the embodiments illustrated below are denoted by the same reference signs as those used in the description of the first embodiment and detailed description of the elements will be omitted as appropriate.

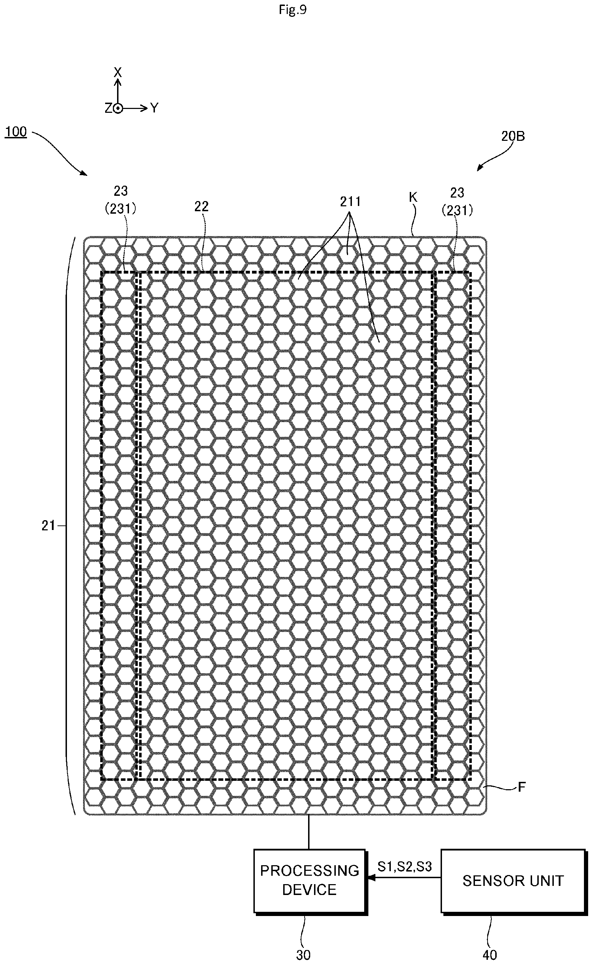

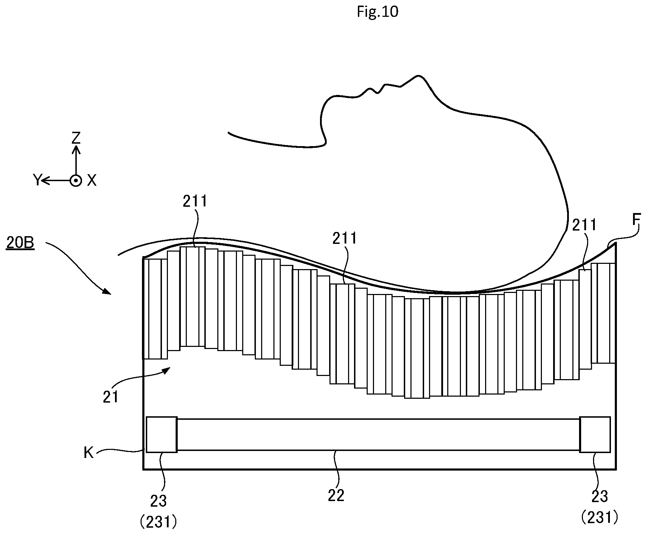

In the bedding system 100 according to the second embodiment, a pillow 20B is illustrated as the bedding. Other components are the same as those of the first embodiment except that the type of bedding is different. Fig. 9 is a plan view of the bedding according to the second embodiment. Similarly to the first embodiment, the bedding system 100 according to the second embodiment also includes the bedding, the auxiliary mechanism 21, the temperature mechanism 22, the moisture mechanism 23, the processing device 30, and the sensor unit 40. Fig. 10 is a side view of the pillow 20B (side view when viewed from the positive side in the X direction). Similarly to the mattress 20A, the pillow 20B also includes the container K. The container K includes a surface (contact surface F) on which the head of the user U is placed when the user U lies down.

As illustrated in Fig. 10, similarly to the first embodiment, the auxiliary mechanism 21, the temperature mechanism 22, and the moisture mechanism 23 are installed inside the pillow 20B. Similarly to the first embodiment, the auxiliary mechanism 21 according to the second embodiment includes a plurality of the column members 211. According to the second embodiment, the diameter of the column member 211 (the length of the diagonal line of the regular hexagon) is, for example, 10 to 20 mm inclusive.

The temperature mechanism 22 of the second embodiment is a mechanism for adjusting the temperature of the pillow 20B. Thus, it is possible to adjust the temperature at the head of the user U. For example, similarly to the first embodiment, the temperature mechanism 22 includes the adjustment liquid and the adjustment device 213. The entire inside of the container K of the pillow 20B is filled. For example, the adjustment liquid may be filled in the gap between the column members 211, the inside of the column member 211, the upper side and the lower side of the column member 211, and the like. In order to move the upper surface of the column member 211 up and down (in and out) in accordance with the shape of the surface of each body part and to support the center of gravity and the body weight load of each body part, a liquid having a large specific gravity is employed as the adjustment liquid so as to realize a buoyancy as large as possible. It is preferable that the temperature mechanism 22 of the pillow 20B is configured to capable of adjusting the temperature for each of a plurality of regions corresponding respectively to a plurality of parts of the head of the user U.

According to the second embodiment, for example, the gap between the column members 211 is filled with the adjustment liquid. Therefore, as illustrated in Fig. 10, the position of the tip of each of the column members 211 follows the shape of the body surface according to the difference in the distribution of the body load due to the buoyancy of the adjustment liquid. As described above, each of the column members 211 moves under the control of the first control unit 321 and also moves in accordance with the load of the user U. A similar configuration can also be employed in the first embodiment.

Similarly to the first embodiment, the moisture mechanism 23 is a mechanism for absorbing or increasing moisture. The moisture mechanism 23 is installed at an arbitrary position inside the container K of the pillow 20B. The moisture mechanism 23 includes a plurality of air blowing devices (for example, compressors or fans) capable of blowing air whose temperature and moisture are adjusted. For example, the air blowing devices may be installed at a position where the blown air hits the head (in particular, the neck) of the user U from the contact surface. Therefore, it is possible to adjust the moisture of the head of the user U by the air blown from the moisture mechanism 23.

Similarly to the first embodiment, the sensor unit 40 of the second embodiment includes the first acquisition unit 41, the second acquisition unit 42, and the third acquisition unit 43.

Similarly to the first embodiment, the first acquisition unit 41 is a sensor used to identify the body position and the change period. The body position of the second embodiment is the position of the head (for example, the center of gravity) of the user U on the contact surface.

Similarly to the first embodiment, the first acquisition unit 41 generates the detection signal S1 that indicates a displacement in accordance with the load of the head of the user U on the contact surface F. Similarly to the first embodiment, the detection signal S1 is used to identify the body position and the change period. For example, a plurality of the first acquisition units 41 are provided at predetermined intervals in a region on the contact surface F side inside the pillow 20B (container K).

Similarly to the first embodiment, the second acquisition unit 42 is a sensor that generates the detection signal S2 representing the body temperature of the user U. The second acquisition unit 42 is mounted (attached) to, for example, a plurality of parts such as the head and the neck of the user U.

Similarly to the first embodiment, the third acquisition unit 43 is a sensor that generates the detection signal S3 representing the moisture of the body surface of the user U. For example, the third acquisition unit 43 may be mounted to the same position as the second acquisition unit 42 on the body surface of the user U.

Similarly to the first embodiment, the first control unit 321 controls the movement of each of the column members 211 of the auxiliary mechanism 21 in accordance with the change period and the body position. First, the first control unit 321 controls the timing at which each of the column members 211 moves in accordance with the change period. Specifically, when the determination unit 311 determines that the change period exceeds the threshold Z, the first control unit 321 causes each of the column members 211 to move. Second, the first control unit 321 controls the position to which each of the column members 211 moves in accordance with the body position.

Similarly to the first embodiment, the second control unit 322 controls the temperature mechanism 22 in accordance with the body temperature of the user U (biological information). Similarly to the first embodiment, the third control unit 323 controls the moisture mechanism 23 in accordance with the moisture of the body surface of the head of the user U.

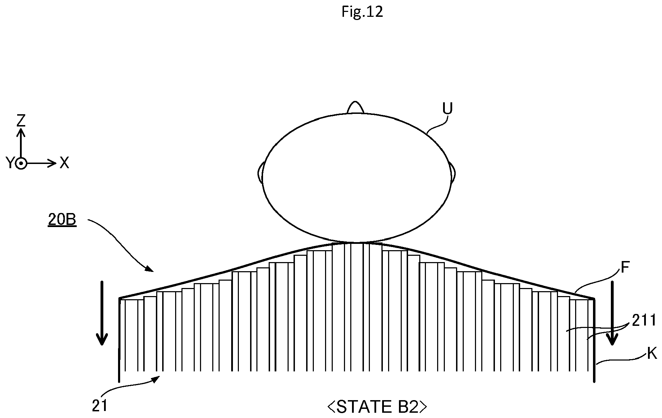

Hereinafter, an example of the movement of the plurality of the column members 211 of the auxiliary mechanism 21 according to the second embodiment will be described. Figs. 12 to 14 are explanatory views illustrating an example of the movement of the column members 211 when viewed from the Y direction. Figs. 12 to 14 show a state of the column members 211 when the user U changes the body position (turns over) from a supine position to a lateral position.

As illustrated in Figs. 12 to 14, for example, the movement of the column members 211 is controlled so as to transition from a state B1 to a state B2 to a state B3 to a state B4. As a result, the user U changes the body position (turns over) from the supine position to the lateral position. The body position is continuously identified from the state B1 to the state B4. The movement of the column members 211 from the state B1 to the state B4 is performed by identifying the body position. The states B1 to B4 correspond to the states A1 to A4 of the first embodiment.

Schematically, the user U is caused to turn over by lowering the tip (the end portion on the positive side in the Z direction) of one or more of the column members 211 on one of the left and right sides of the user U when viewed from the Y direction among the plurality of the column members 211.

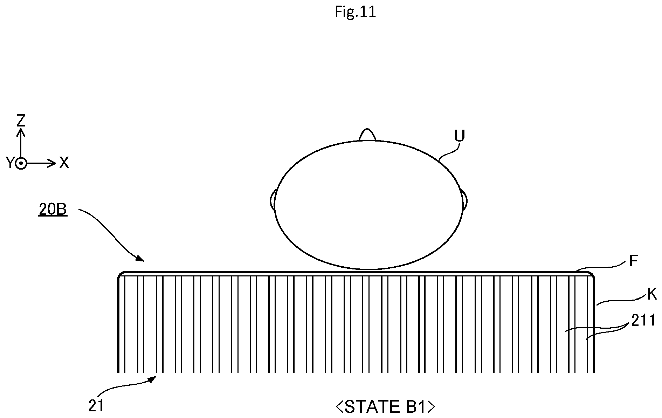

The state B1 in Fig. 11 is a state of the column members 211 when the user U is in the supine position. In the state B2 of Fig. 12, among the plurality of the column members 211, one or more of the column members 211 corresponding to predetermined widths on both the left and right sides (negative side and positive side in the X direction) of the head of the user U are lowered (moved to the negative side in the Z direction). As illustrated in Fig. 12, for example, the column members 211 are moved so as to incline the region from the body position side to the end portion on the positive side and the end portion on the negative side in the X direction. Among the plurality of the column members 211, the column members 211 positioned at the end portion on the positive side and the end portion on the negative side in the X direction have, for example, the tips lowered by about 15 mm as compared with the column members 211 corresponding to the body position. In the state B1, when the determination unit 311 determines that the change period exceeds the threshold Z, the operation of moving the plurality of the column members 211 from the state B2 to the state B4 is executed.

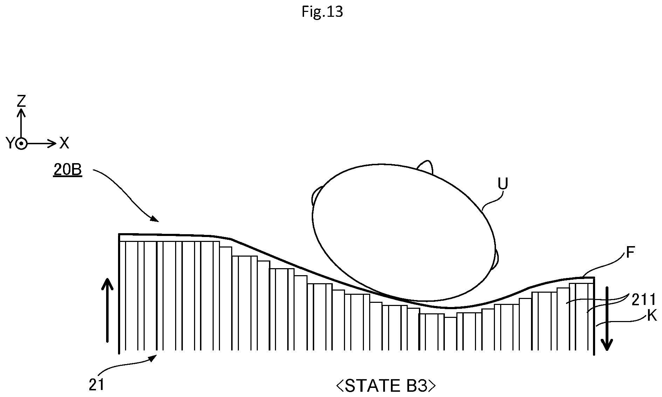

In the state B3 of Fig. 13, one or more of the column members 211 which correspond to the predetermined widths and are positioned on one of the left and right sides of the head of the user U among the plurality of the column members 211 are further lowered. Specifically, after the state B2 is set, the column members 211 on the side to which the head of the user U voluntarily moves are further lowered. Fig. 13 illustrates a case where one or more of the column members 211 positioned on the right side (positive side in the X direction) of the head are further lowered from the state B2. For example, the end portion on the positive side in the X direction is lowered by about 15 mm from the state B2.

On the other hand, one or more of the column members 211 which correspond to the predetermined widths and are positioned on the left side (negative side in the X direction) of the user U are raised from the state B2 (for example, raised by about 15 mm). As long as the end portion on the positive side in the X direction is within 15 mm from the state B2, the distance to be lowered is arbitrary. Similarly, as long as the end portion on the positive side in the X direction is within 15 mm from the state B2, the distance to be raised is arbitrary.

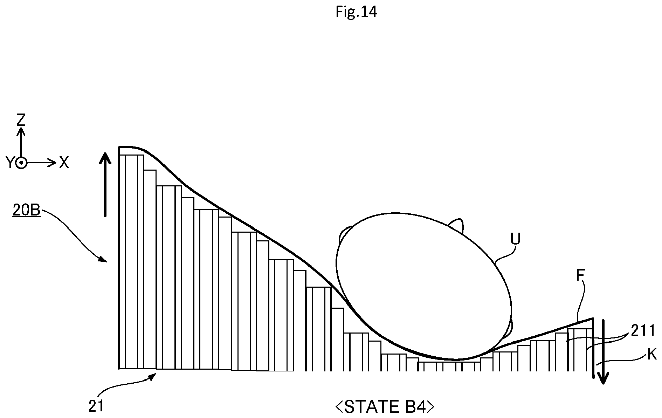

In the state B4 of Fig. 14, from the state B3, one or more of the column members 211 positioned on the right side of the head of the user U are further lowered, and one or more of the column members 211 positioned on the left side of the user U are further raised. For example, in the state B4, from the state B1, one or more of the column members 211 positioned on the right side of the user U are lowered by about 30 to 60 mm inclusive, and one or more of the column members 211 positioned on the left side of the user U are raised by about 30 to 60 mm inclusive. That is, a height difference of about 60 to 120 mm inclusive is generated in the plurality of the column members 211.

Due to the movement from the state B1 to the state B4, among the plurality of the column members 211, one or more of the column members 211 on one of the left and right sides of the user U (the right side in the first embodiment) when viewed from the Y direction move downward, and one or more of the column members 211 on the other side (the left side in the first embodiment) move upward. That is, among the plurality of the column members 211, the inclination from one side to the other side of the user U when viewed from the Y direction has a gentle gradient. Since the body position of the user U is changed in accordance with the movement of the plurality of the column members 211, it is possible to change the body position (turn over) from the supine position to the lateral position.

### <Modification>

The embodiments illustrated above may be modified in various ways. Specific modification aspects will be illustrated below. It is also possible to appropriately combine two or more aspects arbitrarily selected from the following examples.
(1) In each of the above-described embodiments, the change period is used to control the timing at which the column members 211 are moved, but the biological information used to control the timing at which the column members 211 are moved is not limited to the above-described examples. For example, the following various types of information are used to control the timing at which the column members 211 are moved.

### [Information on snoring]

The information on snoring is, for example, the volume of snoring of the user U. The first acquisition unit 41 (for example, a microphone) collects snoring of the user U and generates the detection signal S1 corresponding to the sound. The determination unit 311 identifies the volume of snoring represented by the detection signal S1 and compares the volume with a predetermined threshold. When the determination unit 311 determines that the volume of snoring exceeds the predetermined threshold, the first control unit 321 moves the column members 211. The information on snoring is not limited to the volume of snoring. For example, the frequency of snoring or the like may be used as the information on snoring.

### [Information on breath]

The information on breath is, for example, the breath rate of the user U. The first acquisition unit 41 is any sensor capable of detecting the breath of the user U, and generates the detection signal S1 representing the breath of the user U. The determination unit 311 identifies the breath rate in a predetermined period from the detection signal S1 and compares the breath rate with a predetermined threshold. When the determination unit 311 determines that the breath rate is lower than the predetermined threshold, the first control unit 321 moves the column members 211.

In addition, apnea period may be used as information on breath. The determination unit 311 identifies an apnea period from the detection signal S1, and compares the period with a predetermined threshold. When the determination unit 311 determines that the period exceeds the predetermined threshold, the first control unit 321 moves the column members 211. The information on breath is not limited to the breath rate and the apnea period.

### [Information on heartbeat]

The information on heartbeat is, for example, the heart rate of the user U. The first acquisition unit 41 is any sensor capable of detecting the heart rate of the user U, and generates the detection signal S1 representing the heart rate of the user U. The determination unit 311 identifies the heart rate in a predetermined period from the detection signal S1 and compares the heart rate with a predetermined threshold. When the determination unit 311 determines that the heart rate exceeds (or is lower than) the predetermined threshold, the first control unit 321 moves the column members 211.

In addition, the occurrence of arrhythmia may be used as information on heartbeat. The determination unit 311 detects the occurrence of arrhythmia from the detection signal S1. When the determination unit 311 detects arrhythmia, the first control unit 321 moves the column members 211.

As can be understood from the above description, the biological information used to control the timing at which the column members 211 are moved is arbitrary. The biological information used to control the timing at which the column members 211 are moved is not limited to the above examples. For example, various kinds of information such as body temperature, body motion, and body surface moisture are exemplified as the biological information used to control the timing at which the column members 211 are moved. A plurality of types of biological information may be combined and used to control the timing at which the column members 211 are moved.
(2) The biological information used to control the position to which each of the column members 211 moves is not limited to the body position. For example, various kinds of information such as body temperature, body motion, and body surface moisture are exemplified as the biological information used to control the position to which the column members 211 move. A plurality of types of biological information may be combined and used to control the position to which the column members 211 move. As understood from the above description, the biological information is a general term of information used to control the auxiliary mechanism 21. A sensor corresponding to the type of biological information is appropriately employed as the first acquisition unit 41.
(3) The biological information used to control the temperature mechanism 22 is not limited to the body temperature of the user U. For example, other various types of biological information may be used to control the temperature mechanism 22. Similarly, the biological information used to control the moisture mechanism 23 is not limited to the moisture of the body surface of the user U.
(4) In each of the above-described embodiments, for example, a configuration, in which the user U evaluates the degree of comfortable sleep (for example, 10-level evaluation) at the time of waking up from sleep using the bedding system 100, may be employed. The user U inputs the evaluation using the input device. Then, the second control unit 322 may set the target body temperature in consideration of the evaluation by the user U. Similarly, the third control unit 323 may set the target moisture in consideration of the evaluation by the user U. That is, the second control unit 322 and the third control unit 323 may perform feedback control on the temperature mechanism 22 and the moisture mechanism 23 based on the evaluation by the user U.

In the above-described configuration, the second control unit 322 may set the target body temperature (the target temperature at which it can be estimated that the user U can sleep most comfortably) using a learned model obtained by learning the relation between the degree of comfortable sleep and the target body temperature. A learned model obtained by learning the relation among the degree of comfortable sleep, various other parameters (temperature, body temperature, moisture, psychological state, bowel movement, urination, atmospheric pressure, season, and the like), and the target body temperature may be used. Similarly, the third control unit 323 may set the target moisture (the target moisture at which it can be estimated that the user U can sleep most comfortably) using a learned model obtained by learning the relation between the degree of comfortable sleep and the target moisture. A learned model obtained by learning the relation among the degree of comfortable sleep, various other parameters (temperature, body temperature, moisture, psychological state, bowel movement, urination, atmospheric pressure, season, and the like), and the target moisture may be used.

The learned model is a statistical estimation model generated by machine learning. For example, various statistical estimation models such as decision tree or neural network may be preferably used as the learned model. The learned model is realized by a combination of a program (for example, a program module constituting artificial intelligence software) that causes the control device 31 to execute an operation of generating output data from input data and a plurality of coefficients applied to the operation. The plurality of coefficients are set by machine learning (in particular, deep learning) using a large amount of teacher data and are held in the storage device 32.

For the user U who uses the bedding system 100 of the present invention for the first time, when there is no lesion such as inflammation or chronic fatigue in each body part, the target moisture is set to 50% or less (43% to 50% inclusive), and the target temperature is set to, for example, 26.0°C for the trunk and the four limbs, 25.0°C for the neck, and 18.0°C for the head. Then, the user U evaluates the degree of comfortable sleep at the time of waking up in a case where the target temperature is set to increase at 0.25 to 0.5°C inclusive per hour while the moisture is constantly maintained at the target moisture of 50% or less in each body part over time. As described above, a configuration in which evaluation for each body part is input may also be employed. Here, it is not necessary to control the moisture mechanism 23 and the temperature mechanism 22 for each part.

In addition, the first control unit 321 may control the movement of the column members 211 of the auxiliary mechanism 21 in consideration of the evaluation by the user U. For example, the first control unit 321 may control the movement of the column members 211 using a learned model obtained by learning the relation between the degree of comfortable sleep and the movement (for example, the timing or the position of the movement) of the column members 211.

According to the configuration in which the temperature mechanism 22 and the moisture mechanism 23 are controlled using the learned model, it is possible to individually optimize the target temperature and the target moisture for each user U. Consequently, it is possible to promote comfortable sleep for the user U.
(5) In each of the above-described embodiments, a bedding in which the mattress 20A and the pillow 20B are integrated may be used in the bedding system 100. In a bedding in which a mattress and the pillow 20B are integrated, the components and the control methods of the auxiliary mechanism 21, the temperature mechanism 22, and the moisture mechanism 23 are the same as those in the above-described embodiments.
(6) According to the second embodiment, the bedding is portable (can also be used with a storage battery). The material of the container K of the bedding is washable. Several kinds of sizes from a large size to a portable small size are prepared for the pillow 20B, and in the case of combination with the mattress 20A, the pillow 20B is limited to two kinds of small sizes. In the case of a single body of the pillow 20B, the maximum length of the single body is about 700 to 900 mm inclusive and is longer than from the top of the head to below the scapulae, and the maximum width of the single body is also 700 to 900 mm inclusive, and in the case of a combination or a portable single body, the length is about 250 to 350 mm inclusive which is from the top of the head to above the neck, and the width is about 450 to 600 mm inclusive and exceeds the shoulder width. The height is obtained by subtracting the length from the (rotation) center of the body axis, i.e., the center of the neck to the side of the head from the shoulder width to facilitate turning over from the lateral position (a slightly more width is provided in consideration of the comfort of the individual), and when the user turns over from the supine position, the length from the center of the body axis, i.e., the center of the head to the occipital end is subtracted so that the height is slightly lower than when the user turns over from the lateral position. As described above, as long as the entire weight is within a portable range, a slightly more width is provided according to the physique of an individual.
(7) In each of the above-described embodiments, the auxiliary mechanism 21, the temperature mechanism 22, and the moisture mechanism 23 may be controlled in consideration of the body weight, the height, the age, the gender, the medical history, and the like of the user U.
(8) In each of the above-described embodiments, the first control unit 321 may move the column members 211 at predetermined intervals in accordance with the biological information.
(9) In each of the above-described embodiments, it is preferable that the material of the bedding (in particular, the container K) is a hygroscopic material, and the material is selected with particular attention paid to measures against dew condensation and static electricity caused when the target temperature of the temperature mechanism 22 is lowered.
(10) In each of the above-described embodiments, a method of controlling the auxiliary mechanism 21, the temperature mechanism 22, and the moisture mechanism 23 may be as follows.

### <Mattress 20A>

The second control unit 322 controls the temperature mechanism 22 so that the temperature of the head and neck is 20 to 24°C inclusive, which is below the body temperature by 10°C or more, at the time of falling asleep and 28°C, which is below the body temperature of the trunk by 7°C or less, in the morning when the body temperature rises endocrinally to wake up, based on the knowledge that the coolness of the head due to a comfortable low temperature helps to lower the temperature of the brain, which is overheated during the day, and makes sleep more comfortable.

In addition, the time and content of meals, particularly dinner, are greatly related to comfortable sleep. In addition, a sympathetic excitation state due to coffee intake or smoking after dinner, excessive water intake, intense exercise, or the like may disturb sleep, so caution is required. Recently, it has been known that physiologically active polypeptides including various hormone-like substances and neurotransmitters such as serotonin, which are produced from the intestinal flora of 100 trillion or more, interact with the central nervous system and the immune system inside and outside the intestinal tract. In addition, it has become clear that sleep and intestinal environment (balance between intestinal flora and intestinal immunity) also exert a great influence on each other. As the intestinal flora is dominated by so-called beneficial bacteria, whether or not a sufficient amount of dietary fiber is ingested to feed the bacteria, secretion of various hormones in relation to the biological clock, meal time, sleep time, etc. are also important for comfortable sleep.

In other words, it is well known that although it depends on the degree of obesity, degree of accumulation of visceral fat, diaphragm elevation, presence of hiatal hernia or other hernia, reflux esophagitis is very likely to occur when the time from dinner to falling asleep is as short as 1 to 2 hours and sleep is disturbed by heartburn, coughing, etc. Further, in the case of a meal with a large amount of meat and oil, it takes a long time to digest, and an interval of 3 to 4 hours is required. In the case of a vegetable-based meal, it is considered that the load on the stomach is light and thus the interval can be 2 to 3 hours. Therefore, salad and soup are desirable for late evening dinner. Recently, the relation between intestinal bacteria and sleep has also been considered, and a vegetable-based diet containing sufficient dietary fiber is better in the relation with bowel movement.

From the above point of view, in order to prevent reflux esophagitis and to reduce the cardiac burden, each of the column members 211 of the auxiliary mechanism 21 may be controlled so that the upper body part of the bedding is raised by about 10 to 20 degrees inclusive at the time of falling asleep and the angle is reduced to 0 to 5 degrees inclusive over 1 to 3 hours inclusive after falling asleep depending on the sleeping state including turning over, etc.

The interval between the bathing time and the falling asleep time is also of great significance. That is, after bathing for about 5 to 10 minutes inclusive in a half-body bath at a warm temperature of 39 to 40°C inclusive, it is preferable to go to bed after 1.5 hours, if possible, when the body temperature drops and sleepiness is likely to occur. Such daily contents also need to be input in order to realize comfortable sleep. When the time until falling asleep is short, the initial set temperature of the portion (adjustment liquid) corresponding to the upper half of the body of the temperature mechanism 22 is also lowered than usual when room temperature adjustment is difficult so as to intensify sleepiness.

In addition, it is aimed to completely eliminate nocturnal awakening ranging from 2 to 5 times inclusive and early morning awakening at 1 to 5 a.m. inclusive, which most disturb the feeling of comfortable sleep and tend to cause depressed mood, but it is also a big point whether or not resleeping is possible particularly when nocturnal awakening or early morning awakening occurs due to shift work, etc. Therefore, when the weight of the head or the load of the body weight is not present for, for example, one minute or more, it is regarded as an awake state, and the state is brought close to the state at the time of falling asleep. That is, for example, in the case of being not present for 10 minutes or more, the target body temperature of the temperature mechanism 22 and the target moisture of the moisture mechanism 23 are reset to the state at the time of falling asleep to facilitate resleeping. In addition, when falling sleep cannot be achieved smoothly, the starting temperature setting is set to be lower than the initial setting by 1 to 2°C or more, for example, 20 to 22°C inclusive for the head and neck, 22 to 24°C inclusive for the chest, and 24 to 26°C inclusive for the lower body and the temporal changes of the target body temperature and target moisture until the scheduled wake-up time are reset to realize comfortable sleep and comfortable wake-up at the initially set wake-up temperature when waking up.

In order to make the wake-up comfortable, the temperature of the temperature mechanism 22 is raised toward the scheduled wake-up time, and at the same time, the light and the sound of the indirect lighting which brightens with the lapse of time from 30 to 60 minutes inclusive before the wake-up by the lighting device provided on the side surface of the bedding system 100 also promote the wake-up, and meanwhile, the acoustic equipment provided in the bedding system 100 also functions as an alarm clock with a sound that does not cause discomfort. Further, when the user does not get up after the scheduled time (when the body weight load on the bedding does not disappear), the vibration device which vibrates harder with time is also activated to promote the user to get up.

### <Pillow 20B>

First, for comfortable sleep, it is not only to fall asleep easily, but also to secure at least 20-25% of deep sleep (percentage of NREMst3), to be able to turn over easily, and to wake up naturally when getting up, and the following factors are also important: a feeling of recovery from fatigue, a feeling of comfortable sleep and satisfaction, and a positive feeling toward work without a heavy head, headache, pain in various parts of the body, fever, fatigue, malaise, or mental or psychological depressive tendencies.

As understood from the above description, the target body temperature and the target moisture are not always constant values, but may be changed over time or may be changed due to various factors.

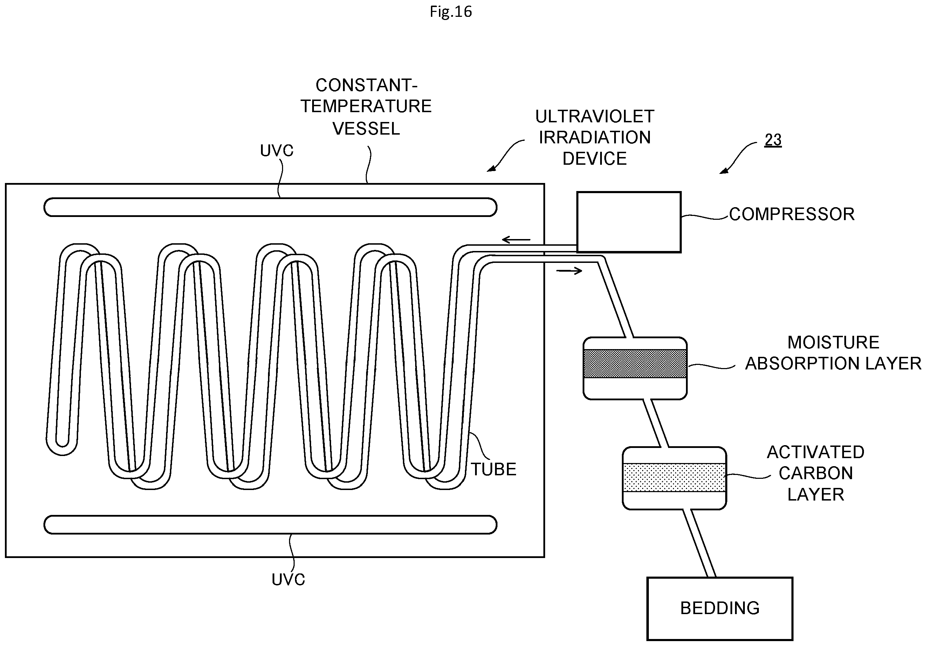

(11) It is necessary to take measures against microbial infections caused by viruses, bacteria, fungi, etc. which are not limited to the measures against COVID-19. Therefore, in the moisture mechanism 23 according to the modification, a configuration related to measures against infections is employed. Fig. 16 is a configuration diagram of the moisture mechanism 23 according to the modification. As illustrated in Fig. 16, the moisture mechanism 23 includes a compressor, an ultraviolet irradiation device, a moisture absorption layer, and an activated carbon layer. The air blown from the compressor is blown to a bedding 20 (20A, 20B) via a tube. The ultraviolet irradiation device, the moisture absorption layer and the activated carbon layer are provided between the compressor and the bedding.

The air blown from the compressor (for example, at a wind velocity of 0.05 to 0.1 m / sec inclusive) first passes through the ultraviolet irradiation device, and the ultraviolet irradiation device includes, for example, a constant-temperature vessel and a UVC lamp (germicidal lamp). The tube is arranged in the constant-temperature vessel set to a predetermined temperature (for example, it can be set to 18°C to 28°C inclusive in increments of 1°C), and the tube is irradiated with the UVC lamp. For example, the irradiation by the UVC lamp is performed for 10 seconds or more during which sufficient killing effect on microorganisms such as viruses and bacteria is exhibited. In addition, in the ultraviolet irradiation device, the moisture can be set to a predetermined moisture (for example, can be set in increments of 2% in a range of 40% to 52% inclusive).

The air that has passed through the ultraviolet irradiation device further passes through the moisture absorption layer (for example, silica gel) whose moisture can be adjusted, and is blown to the activated carbon layer. The activated carbon layer collects ozone generated in the ultraviolet irradiation device. Then, the air that has passed through the activated carbon layer is blown to positions corresponding to respective parts of the bedding. The moisture absorption layer is not indispensable.

Further, the ultraviolet irradiation device may be provided for each body part. The moisture mechanism 23 may be entirely provided inside the bedding or may be partially provided outside the bedding. Fig. 16 illustrates a configuration in which the moisture adjustment device includes one ultraviolet irradiation device, but the number of ultraviolet irradiation devices is arbitrary and can be appropriately changed in accordance with the length of the tube. Regardless of the number of ultraviolet irradiation devices, from the viewpoint of obtaining a sufficient killing effect, a configuration that allows irradiation with the UVC lamp for 10 seconds or more as a whole is preferable.

The temperature and moisture in the moisture mechanism 23 are expected to change before reaching each body part. Therefore, the relation between the temperature and moisture in the moisture mechanism 23 and the temperature and moisture at each body part may be learned by machine learning in a learned model for each air volume and wind velocity in consideration of the outside air temperature, moisture, weather, the body temperature of the user, and the physical condition including inflammation at each body part, etc. The temperature and moisture in the moisture mechanism 23 may be set using the learned model.

(12) In the bedding system 100 according to each of the above-described embodiments, it is possible that the movement of the external auxiliary mechanism 21 (the column members 211), which blocks the excessively large spontaneous movement of the user himself / herself, disturbs comfortable sleep. First, in order to investigate the user U's spontaneous movement of body axis, center of gravity, or breath, etc., the user's limbs, body movements, turning over, etc. under the air pressure, temperature, moisture, etc. are monitored and recorded over time under any initial target temperature and target moisture settings for one (normal) to two (in the case of shift work, etc.) weeks after use in a flat state to monitor and record over time various parameters such as body weight, heartbeat, breath, snoring, change of body temperature, etc., and temporal, physical, or geographical changes in sleeping posture, body movement, and turning over are firmly recorded and grasped as temporal changes on the three-dimensional coordinate axis, and the recorded information is utilized for conditions at the next use or realization of induced turning over, etc.

In addition, before use and during use for 1 (normal) to 2 (in the case of shift work, etc.) weeks, the user checks the attached comfortable sleep checklist, and meanwhile, the user's feeling of comfortable sleep and satisfaction, etc. is fed back, the target temperature and target moisture are set to the comfort mode over time, the user's limbs, body movements, turning over, etc. are monitored and recorded over time to monitor and record various parameters such as body weight, breath, snoring, body temperature change, etc. over time, and temporal, physical, or geographical changes in sleeping posture, body movement, and turning over are recorded and grasped as temporal changes on the three-dimensional coordinate axis, and the recorded information is utilized for an initial condition at the time of full-scale use, or for an aid in changing the body position. As the comfortable sleep checklist, for example, the treatment may be changed in a plurality of stages as follows.
Score of 8 or less: leave as it is
Score of 9 to 14 inclusive: require adjustment by equipment personnel
Score of 15 to 20 inclusive: require instructions such as change of prescription from an attending physician
Score of 21 or higher: require treatment by a sleep specialist or neuropsychiatrist

(13) In each of the above-described embodiments, the auxiliary mechanism 21, the moisture mechanism 23, and the temperature mechanism 22 may be controlled in consideration of the following various kinds of information at the time of description before use.

Family members living with the user (including whether or not there is stress, caregiving status, etc.), height and body weight (BMI), medical history (especially stroke and heart disease such as arrhythmia), complications (including nasal and oral diseases such as chronic tonsillitis and sinusitis), current illness (including hypertension, diabetes, heart diseases such as heart failure, cerebrovascular disease, asthma, emphysema, liver disease, depression, etc.), family history, life history (sleeping habits (sleeping posture (mainly supine position, right lateral position, left lateral position), sleeping time (including naps and repeated naps, etc.)), bathing (including time, temperature, sauna, etc.), coffee (including time (daytime, after evening), quantity, etc.) and other addictive products including smoking (past smoking, current smoking or passive smoking) and drinking (frequency, quantity, whether or not red face appears), allergy history (including hay fever and asthma), usual blood pressure, pulse rate, and body temperature, recent symptoms, usual meal times, meal contents, whether or not the user eats between meals (contents), work contents (whether or not there is stress (interpersonal relationships, etc.)) and work hours (including whether or not the user works at night, works in shifts, etc.), and the AHI value at the time of sleep apnea syndrome diagnosis, etc. are to be entered by the user U using the input device.

Further, if possible, the user is prompted to input the physical condition of the day, the stress at work, the time and content of exercise, the time and content of meal, the presence or absence and amount of drinking, the time and content of bathing, and the like during the time from dinner to before sleeping every day. After getting up, the user U is also prompted to input the sleep time and content (including the number of times of waking up and the number of night-time toilet visits, etc.), the physical condition at the time of getting up, feeling of comfortable sleep or symptoms such as headache and dryness, the condition or malfunction of the device, and the like in the same manner.

In addition, better comfort can be pursued in units of 1 to 4 weeks inclusive or by reviewing such as comparison with the conventional average or the previous week or period. Furthermore, as items related to subjective symptoms, the user is prompted to input conditions of daytime sleepiness (e.g., almost always doze off when: sitting to read newspaper or book, sitting to watch TV, sitting quietly in a conference or at a cinema or theater, sitting for one hour in a car driven by others, lying down to rest in the afternoon, sitting to talk with a person, sitting quietly without drinking after lunch, driving by the user himself / herself, stopping for several minutes due to traffic jam, etc.), and whether or not there are the following symptoms. The user is also prompted to input information such as frequently snoring, waking up with difficulty in breathing, waking up with a desire to urinate two or more times in the night, feeling a decrease in memory and concentration, feeling tired and unable to get rid of fatigue, cessation of breathing or apnea for several minutes during sleep noted by family members, sleeping lightly at night or frequent waking up, heavy head or headache at the time of getting up or in the morning, feeling chronic sleep deprivation without a feeling of deep sleep upon getting up, and drinking in place of hypnotics every day.
(14) The bedding is not limited to the mattress 20A and the pillow 20B. For example, the present invention may be applied to a chair-type bedding. Examples of the chair-type bedding include, for example, a reclining chair, a massage chair, and chairs for users with various diseases (for example, disabled persons, severe obesity persons, sarcopenia, and the like).
(15) Sweating observed with the lapse of time during sleep increases the body surface moisture and lowers the body surface temperature. Therefore, temperature and moisture during sleep is required to be controlled in accordance with the elapse of time in consideration of psychosomatic states such as changes in body temperature, moisture, and perspiration of each body part.

In consideration of the above circumstances, in the bedding system 100, it is preferable to employ a configuration in which the second control unit 322 controls the temperature mechanism 22 so that the temperature of the bedding (in particular, the mattress 20A) changes over time while the user U is sleeping. In the above-described configuration, it is preferable that the bedding system 100 includes any known sensor that determines whether or not the user has fallen asleep and measures the time elapsed after falling asleep.

Specifically, before falling asleep, the second control unit 322 lowers the temperature of the temperature mechanism 22 so as to lower the core body temperature of the trunk as well as the head and neck so that the excited state of sympathetic nerves is suppressed and the user can fall asleep smoothly. On the other hand, when the core body temperature of the trunk continues to decrease during the sleep period, the body is in a hypothermic state, causing functional deterioration of internal organs and tissues, etc., and there is a possibility that fatigue recovery cannot be achieved and a feeling of malaise or fatigue is felt after getting up or a fresh and positive feeling is not felt. Therefore, the second control unit 322 gradually increases the temperature of the temperature mechanism 22 over time from after falling asleep to before getting up. The increase in the core body temperature also results in a gradual increase in the temperature of blood flow to the brain. However, in order to prevent heavy head, headache, and the like and to obtain a clear feeling, it is preferable to raise the temperature of the head and neck, for example, 30 minutes before getting up to reliably ensure a comfortable sleep.

In the above configuration, it is more preferable to individually control the temperature of a portion of the bedding corresponding to the head and neck parts and a portion of the bedding corresponding to the trunk part (the part below the neck part). The second control unit 322 controls the temperature mechanism 22 so that the temperature of a portion (region) of the bedding corresponding to the body part changes such that the above-described core body temperature of the user U changes over time. In addition, the second control unit 322 controls the temperature mechanism 22 such that the portion of the bedding corresponding to the head and neck parts does not significantly reduce the brain function and the brain circulation and the user can sufficiently obtain deep sleep (stage 3 of non-REM sleep).

The second control unit 322 may control the temperature mechanism 22 in accordance with the depth of sleep of the user U. For example, the second control unit 322 controls the temperature mechanism 22 such that the core body temperature decreases and deep sleep (stage 3 of non-REM sleep) is increased to improve the balance of the autonomic nerve at the time of nocturnal awakening or early morning awakening due to nighttime urination, etc. or when the user U is in the stages 1 and 2 of non-REM sleep or in the REM sleep state one hour or more before the scheduled time of getting up after falling asleep, and when the user U is in the REM sleep state within one hour before the scheduled time of getting up, the second control unit 322 controls the temperature mechanism 22 so that the core body temperature rises.

In the above configuration, it is preferable that the bedding system 100 includes a sensor that detects the sleep state of the user U or the user enters the level of comfortable sleep when getting up to give feedback and let AI learn and improve the configuration. Since it is known that most of the growth hormones secreted during the deep sleep period within 3 hours after falling asleep play important roles in maintenance of internal organs and maintenance of hair, it is necessary to ensure the deep sleep period within 3 hours after falling asleep.

In addition to controlling the temperature mechanism 22 over time, the second control unit 322 may control the temperature mechanism 22 according to individual circumstances such as the mental and physical state of the user U before sleeping (for example, environment such as work and home, overwork, stress, and the like), the living state (for example, bathing, exercise, whether or not an addictive product is ingested, and the like), and whether or not the user U has a disease.

Similarly, in the bedding system 100, it is preferable to employ a configuration in which the third control unit 323 controls the moisture mechanism 23 so that the moisture of the bedding changes over time while the user U is sleeping.

(16) The following configuration may be employed in the bedding system 100 from the viewpoint of lowering the core body temperature necessary for falling asleep and naturally turning over because the temperature control of the trunk and the four limbs is insufficient only by the temperature control of the head and the brain.

The sensible temperature is greatly related to moisture. Specifically, the temperature and moisture that change with the elapse of time during sleep greatly depend on the amount of perspiration of the user himself / herself (affected by the perspiration capacity, the amount of water ingested, the amount of exercise, and the like) as well as the external moisture. Therefore, in order to realize comfortable sleep, it is preferable to control moisture, wind, and the like together with temperature in addition to control of temperature during sleep.

The sensible temperature of each body part, the moisture around the body, and turning over during sleep also vary depending on the mental and physical conditions during the day, physical activity, diet, moisture intake, the temperature and moisture of the environment stayed, and the like. Therefore, it is preferable to use AI for controlling the temperature and moisture during sleep. Further, it is more preferable that feedback control is performed using information on whether or not a feeling of comfortable sleep is obtained.

In addition, the progress of sweating related to the change in moisture with the sleeping time is also affected by the temperature and moisture of the bedroom, the amount of exercise in the daytime, the dietary calorie intake, the water intake, the number of times and amount of drinking coffee, the physical condition, and the like.

Furthermore, temperature and moisture can also make a difference in obesity-related heat retention due to subcutaneous fat thickness and moisture changes due to perspiration.

Comfortable sleep requires falling asleep smoothly in a short period of time, as few night-time toilet visits as possible, no nocturnal awakening / early morning awakening, smooth turn-over without spontaneous wake-up such as once every 20 to 30 minutes, no nightmares, comfort and freshness upon natural getting-up, no tiredness / fatigue, headaches / feeling of heavy head upon getting-up, no myalgia, stiff shoulders, low back pain or the like, a positive feeling, etc.

Conditions necessary and sufficient for comfortable sleep, such as temperature, moisture, heights of the column members 211, and differences in height between adjacent columns or among regions, are stored for each natural environment such as season, weather, and atmospheric pressure, for each mental and physical situation of individuals, or for each lapse of time before falling asleep and after falling asleep. Then, by reproducing the stored condition, whether or not comfortable sleep can be obtained is re-detected by inputting a subjective feeling of comfortable sleep, etc., and by storing a condition for further enhancing the feeling of comfortable sleep and reproducing it when comfortable sleep is necessary, etc., reliable comfortable sleep is realized regardless of the area, season, weather, atmospheric pressure, mental and physical conditions of an individual, etc.
(17) It is also preferable that the bedding system 100 according to the present invention is used by a bedridden user, for example. By allowing the user to turn over using the bedding system 100, it is also possible to prevent acne, economy class syndrome, and the like.
(18) In each of the above-described embodiments, a movement mechanism (for example, a hydraulic pump) that moves each of the column members 211 in the vertical direction and the lateral direction may be provided. In particular, when the user is extremely obese with a heavy weight or suffers from sarcopenia, it is preferable to provide a movement mechanism. In a configuration in which a movement mechanism is provided, the position of each of the column members 211 in the vertical direction and the lateral direction is adjusted by the movement mechanism to change the shape of the bedding 20, and thus, it is possible to assist in getting up from sleep, that is, from a lying position to a sitting position and further to a standing position, in addition to smooth turning over leading to better comfortable sleep and correction of a posture leading to light sleep during sleep, for example, a stooped posture.

For example, when the bedding is the mattress 20A, the shape is changed as follows. By adjusting the position of each of the column members 211 in the vertical direction and the lateral direction, the center of gravity portion (hip portion) of the mattress 20A is recessed, the direction of the body is changed from the long axis direction of the mattress 20Ato a direction at a right angle to the below-described bed leaving portion, and meanwhile, getting up to a sitting position is facilitated and the portion of the mattress 20A from the center of gravity to the portion away from the mattress 20A (e.g., in the direction of the wheelchair or caregiver's waiting area) is recessed, transforming the mattress 20A into a chair-like shape with handrails. Therefore, it is possible to facilitate the getting-up of the user. Consequently, it is possible to reduce the burden on the caregiver.

Conversely, when moving from the standing position to the mattress 20A, the mattress 20A is deformed into a chair-like shape with handrails to reduce the burden of assistance, and the center of gravity portion (hip portion) is further recessed, and the body is directed to the long axis direction of the mattress 20A. Therefore, it is possible to reduce the burden of assistance during sleep.

(19) It is also preferable that the bedding system 100 according to the present invention is used to promote comfortable sleep of the user U who is in an extreme environment such as a spacecraft or a deep sea submarine.

### Industrial Applicability

Bed also plays a significant role in sleep quality and quantity. Waterbeds, once popular in the United States, use water pressure to bear the body's weight load relatively evenly over a wide contact area, regardless of the body's tendency to be obese, although it may sink somewhat. In addition, the specific heat of water is relatively high, so waterbeds also have advantages for sleep such as absorbing body heat and reducing temperature changes. However, the total weight is large, and recent findings have revealed that when the mattress is nearly flat, a waterbed may conversely become a burden on breathing during sleep in the case of circulatory failure, respiratory failure, or the like, and thus, medical beds such as smart beds that raise the upper half of the body are mainly used, and are also employed as some home beds. However, as for surface correspondence, such a medical bed is far from the shape of the body surface, and it is difficult to ideally distribute the load of the body weight.

It has been also known that the body temperature during sleep differs from body part to body part, as has been conventionally referred to as cold head and warm feet. This cannot be achieved with a water bed or a smart bed. Furthermore, it is also known that an inflammation site is accompanied by an increase in temperature, which disturbs sleep. As a result of recent whole body MRI examination analysis, it has been found that not only blood flow is increased in a part where brain function is active, in a part other than the brain, digestion and absorption in the gastrointestinal tract, etc. are also accompanied by increased blood flow and increased body temperature.

Similar to waterbeds, it is difficult to change the temperature distribution of each body part in a recent bed mainly made of urethane foam. A hexagonal prism, which is found in a honeycomb structure employed in some beds, as found in a honeycomb, is a very excellent shape in terms of durability and load-bearing capacity. Temperature and moisture control is often difficult for other types of bedding as well.

On the other hand, although turning over is necessary for preventing economy class syndrome, bedsore and the like, turning over cannot be freely performed in cases of severe obesity, sequelae of stroke, spinal disorders such as cervical spondylosis, muscular diseases such as muscular atrophy, sarcopenia (muscle loss), frailty and the like. In addition to the ease with which the user falls asleep, the sleeping posture and the frequency of turning over are also largely affected by individual differences and environmental factors such as ambient light, sound, and vibration, and there is also a theory that many people sleep in supine position and turn over once every 20 minutes. Especially in the case of severe obesity and frailty, some people sleep in a 100% supine position when they lack the muscle strength necessary for turning over naturally. In some cases, such as a case where the user has an underlying disease, nearly 50% of the sleep is in a lower right lateral position or a left lateral position. Turning over may also require extra force when the pillow sinks under the weight of the head. Therefore, it is necessary to select a material which supports the head and the neck widely with a necessary and sufficient surface, does not sink, and has a relatively large specific heat.

### Reference Signs List

- 20A: Mattress
- 20B: Pillow
- 21: Auxiliary mechanism
- 22: Temperature mechanism
- 23: Moisture mechanism
- 30: Processing device
- 31: Control device
- 32: Storage device
- 40: Sensor unit
- 41: First acquisition unit
- 42: Second acquisition unit
- 43: Third acquisition unit
- 100: Bedding system
- 211: Column member
- 311: Determination unit
- 321: First control unit
- 322: Second control unit
- 323: Third control unit
- F: Contact surface
- K: Container
- S1: Detection signal
- S2: Detection signal
- S3: Detection signal
- T: Tube
- U: User

## Claims

1. A bedding system comprising:
a bedding;
an auxiliary mechanism that is incorporated in the bedding and is to assist change of a body position of a user, the auxiliary mechanism including a plurality of column members that are long along a thickness-wise direction of the bedding, are individually movable along the thickness-wise direction, and are arranged to be parallel to each other; and
a first control unit that controls movement of each of the column members.

2. The bedding system according to claim 1, wherein
the first control unit controls movement of each of the column members in accordance with biological information of the user.

3. The bedding system according to claim 2, wherein
the biological information is related to change of the body position, snoring, breath, and heartbeat of the user, and
the first control unit controls a timing at which each of the column members moves in accordance with the biological information.

4. The bedding system according to claim 2, wherein
the biological information is a position of a user with respect to the bedding, and
the first control unit controls a position to which each of the column members moves in accordance with the biological information.

5. The bedding system according to any one of claims 1 to 4, the bedding system further comprising:
a temperature mechanism that is incorporated in the bedding and is to adjust a temperature of the bedding; and
a second control unit that controls the temperature mechanism in accordance with a body temperature of the user.

6. The bedding system according to claim 5, wherein
the second control unit controls the temperature mechanism such that the body temperature of the user is maintained at a target body temperature.

7. The bedding system according to claim 5, further comprising:
a temperature mechanism that is incorporated in the bedding and is to adjust the temperature of the bedding; and
a second control unit that controls the temperature mechanism such that the temperature of the bedding changes over time while the user is sleeping.

8. The bedding system according to any one of claims 1 to 7, further comprising:
a moisture mechanism that is incorporated in the bedding and is to absorb or increase moisture; and
a third control unit that controls the moisture mechanism in accordance with a moisture of a body surface of the user.

9. The bedding system according to claim 8, wherein
the third control unit controls the moisture mechanism such that the moisture of the body surface of the user is maintained at a target moisture.

10. The bedding system according to any one of claims 1 to 8, wherein
the bedding is a mattress, a pillow, or a bedding that integrates a mattress and a pillow.

11. The bedding system according to any one of claims 1 to 9, wherein
the column member is a regular hexagonal prism.

12. The bedding system according to any one of claims 1 to 10, wherein the column member is tubular.
